(19) **Europäisches Patentamt European Patent Office Office européen des brevets**

(11) **EP 4 159 187 A1**

(12) **DEMANDE DE BREVET EUROPEEN**

(43) Date de publication:
**05.04.2023 Bulletin 2023/14**

(21) Numéro de dépôt: **22209693.5**

(22) Date de dépôt: **16.04.2019**

(51) Classification Internationale des Brevets (IPC):
*A61K 8/06* (2006.01)     *A61K 8/73* (2006.01)
*A61K 8/34* (2006.01)     *A61K 8/37* (2006.01)
*A61K 8/39* (2006.01)     *A61K 8/60* (2006.01)
*A61Q 19/00* (2006.01)

(52) Classification Coopérative des Brevets (CPC):
**A61K 8/068; A61K 8/342; A61K 8/375; A61K 8/39; A61K 8/60; A61K 8/604; A61K 8/73; A61K 8/732; A61K 8/738; A61Q 19/00;** A61K 2800/52

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.04.2018 FR 1853362**

(62) Numéro(s) de document de la (des) demande(s) initiale(s) en application de l'article 76 CBE:
**19772775.3 / 3 781 117**

(71) Demandeur: **ROQUETTE FRERES**
**62136 Lestrem (FR)**

(72) Inventeurs:
  • **MENTINK, Léon**
    **59800 LILLE (FR)**
  • **WILS, Daniel**
    **59190 MORBECQUE (FR)**
  • **LHERITIER, Anne-Marie**
    **95000 CERGY (FR)**
  • **PIOT, Sophie**
    **75017 PARIS (FR)**
  • **LAVARDE, Marc**
    **95000 CERGY (FR)**

(74) Mandataire: **Plasseraud IP**
    **66, rue de la Chaussée d'Antin**
    **75440 Paris Cedex 09 (FR)**

Remarques:
Cette demande a été déposée le 25-11-2022 comme demande divisionnaire de la demande mentionnée sous le code INID 62.

(54) **COMPOSITION ÉMULSIFIANTE À USAGE COSMÉTIQUE, POUR L'OBTENTION D'UNE ÉMULSION DE PICKERING H/E, ET PROCÉDÉ DE FABRICATION D'UNE TELLE ÉMULSION**

(57)     La demande a pour objet une composition émulsifiante, notamment à usage cosmétique, apte à permettre l'obtention d'une émulsion de type huile dans eau (H/E), comprenant d'au moins une cyclodextrine, et au moins un émulsifiant H/E d'origine naturelle, ledit émulsifiant présentant une balance Hydrophile-Lipophile (HLB) supérieure à 8. L'émulsifiant H/E d'origine naturelle peut être choisi parmi les alkyl polyglucosides, les mélanges d'au moins un alkyl polyglucoside et d'au moins un alcool gras, et les esters gras non éthoxylés de polyols.

La demande a aussi pour objet une composition de type émulsion de Pickering, notamment à usage cosmétique, contenant la composition émulsifiante selon l'invention, ainsi que le procédé de fabrication, notamment à froid, d'une telle composition de type émulsion de Pickering H/E.

**EP 4 159 187 A1**

**Description**

La présente invention est relative à une composition émulsifiante d'origine végétale prête à l'emploi et directement utilisable à froid qui trouve des applications notamment dans le domaine des cosmétiques. Cette composition se suffit à elle-même et permet directement de stabiliser une émulsion sans l'ajout nécessaire d'un co-émulsifiant d'origine pétrochimique. Cette composition émulsifiante permet en particulier la réalisation facile d'émulsions H/E très fines avec des textures variées, très compatibles avec la peau et présentant de plus, un toucher sec et frais, soyeux et non gras même avec une teneur élevée en phase grasse dans l'émulsion. Cette composition permet aussi de réduire avantageusement la quantité d'émulsifiant d'origine naturel requis pour obtenir une émulsion H/E.

Une émulsion est une dispersion d'un liquide (ou d'une matière rendue liquide) en fines gouttelettes dans un autre liquide non miscible au premier. Elle présente un aspect macroscopiquement homogène mais apparaît hétérogène au microscope. Le liquide sous forme de gouttelettes est appelé phase dispersée (ou discontinue), tandis que l'autre liquide est appelé phase dispersante (ou continue). En général, une émulsion est composée d'eau et d'huile et de deux phases (émulsion simple): une phase hydrophile (aqueuse) et une phase lipophile (grasse). Les émulsions les plus couramment rencontrées sont des émulsions comprenant une phase lipophile dispersée dans une phase aqueuse continue et sont appelées émulsions huile dans eau (H/E), par opposition aux émulsions eau dans huile (H/E).

De nombreuses compositions cosmétiques sont des émulsions, typiquement des émulsions simples, stabilisées par des tensioactifs, aussi appelés émulsifiants. A titre d'exemple, le document EP 0 685 227 propose un système très complexe de compositions cosmétiques antisolaires, comprenant une phase continue aqueuse, un système protecteur capable de filtrer les rayons UV, un tensioactif, des solvants organiques (alcools et polyols inférieurs) et au moins un polymère ou plus particulièrement un copolymère réticulé (acrylates d'alkyle, acétate de vinyle).

Le document FR 2 858 777 décrit quant à lui une émulsion huile dans l'eau contenant au moins un corps gras (esters d'acides gras, les cires, les beurres, les huiles naturelles -végétale, animale, d'origine marine-, synthétiques ou minérales, les huiles hydrogénées et leurs mélanges), au moins un tensioactif (les esters d'acides gras de polyglycérol éthoxylés, les éthoxylats d'alcools), au moins un co-tensioactif et de l'eau.

Le document DE 102 39 647 A1 décrit quant à lui des émulsions huile/eau (H / E) cosmétique et /ou dermatologique contenant (a) une phase aqueuse, (b) une phase huileuse de 0,5 à 10% en poids d'huile (s) non polaire à moyennement polaire avec une polarité de 20- 60 mN / m, (c) 0,005 à 10% en poids de dérivé (s) de cyclodextrine et éventuellement d'autres agents cosmétiques et / ou dermatologiques, adjuvants et additifs.

Le document EP 3 037 083 A1 décrit quant à lui une composition de protection solaire sans émulsifiant.

Le document FR 2 823 438 A1 décrit quant à lui une émulsion huile dans eau, à base d' association auto-émulsionnable d'alcools gras et de glucolipides dans un rapport en poids compris entre 10:1 et 4:1, et elle se distingue en ce qu'elle comprend en outre un agent viscosant dans la phase grasse.

Cependant, l'utilisation de tensioactifs dans des produits destinés à être appliqués chez l'homme ou l'animal, que ce soit sous forme topique, orale, ou autre, peut être problématique. En effet, les tensioactifs peuvent endommager les membranes cellulaires. Ainsi, des efforts sont consentis, notamment dans le domaine cosmétique, pour réduire les effets potentiellement nocifs des tensioactifs, voire éliminer le recours aux tensioactifs.

Aussi, un des buts de la présente invention est de fournir une composition émulsifiante, qui est apte à former une émulsion stable par ajout d'huile ou de corps gras lorsqu'elle mise en présence d'eau, en particulier selon un procédé dit « à froid ».

Avantageusement, cette composition émulsifiante selon l'invention permet de réduire significativement l'emploi de tensioactifs d'origine pétrochimique et non biodégradables, notamment les dérivés de glycols et les dérivés éthoxylés ou polyéthoxylés. Selon une autre variante, elle permet de supprimer le recours à ces tensioactifs d'origine pétrochimique.

De ce point de vue, la composition selon la présente invention permet également de fabriquer des émulsions de type Pickering. Les émulsions de ce type sont dépourvues de tensioactif et sont stabilisées par des particules colloïdales comme d'ordinaire des silices qui se placent aux interfaces de la phase continue et de la phase dispersée. Dans le cadre de la présente invention ces particules colloïdales sont des particules organiques constituées de complexes d'inclusion entre au moins une cyclodextrine et au moins une molécule grasse. Ces particules sont très avantageusement compatibles avec la peau ou les cheveux et n'endommagent pas les membranes cellulaires de nature animale.

Ce résultat est d'autant plus remarquable que l'état de la technique démontre que la fabrication de systèmes émulsifiants à usage cosmétique et contenant des cyclodextrines, ne permet pas d'obtenir des émulsions facilement et directement à froid : il était nécessaire jusqu'alors de recourir à des tensioactifs classiques fonctionnant à haute température et généralement d'origine pétrolière. C'est notamment ce que relate le document EP 2 091 502 B1 qui décrit une émulsion H/E contenant de l'eau, une substance grasse, un polysaccharide modifié, et une cyclodextrine, la caractéristique essentielle de cette émulsion H/E étant qu'elle contient des agents tensioactifs de poids moléculaire inférieur à 5 000 g/mol et en quantité inférieure à 2 % en poids. Il n'était donc pas connu, ni évident, de réaliser des systèmes émulsifiants contenant des cyclodextrines permettant d'obtenir des émulsions très fines et très stables et ce, sans avoir recours à des tensioactifs ordinaires d'origine pétrochimique.

Un autre objet de la présente invention est de fournir une composition d'origine naturelle. L'origine naturelle des ingrédients servant à formuler des produits d'usage courant comme des compositions cosmétiques est aujourd'hui un enjeu majeur, non seulement vis-à-vis de la sauvegarde et de la protection de notre environnement mais aussi pour le bien être des consommateurs. A ce titre, la composition selon la présente invention permet de réduire, voire de remplacer, les émulsifiants traditionnels, notamment éthoxylés, qu'on cherche de nos jours à substituer pour des questions environnementales (mauvaise biodégradabilité) et de sécurité (l'oxyde d'éthylène est toxique et inflammable).

Un autre objet de la présente invention est de fournir une composition émulsifiante prête à l'emploi, permettant au formulateur une mise en œuvre très simple, avec un apport minimal d'énergie notamment par introduction de tous les ingrédients dans une même cuve ou réacteur (formulation dite « one pot » en langage anglo-saxon). Du point de vue de sa mise en œuvre, la composition objet de la présente invention est avantageusement utilisable selon « un procédé à froid », c'est-à-dire même à température ambiante, contrairement à bon nombre d'émulsifiants conventionnels solides ou pâteux tels que les cires qui requièrent une élévation de la température en vue de leur mise en œuvre (ingrédient nécessitant d'être fondu et utilisable « à chaud »). La notion de procédé à froid inclue les procédés d'émulsification dans lesquels le seul apport de chaleur est celui dû à la dissipation d'énergie causée par une agitation mécanique. Par « procédé à froid », on entend que la composition émulsifiante peut être mise en œuvre directement par dispersion dans l'eau à une température d'eau inférieure à 45°C, mieux inférieure à 35°C, et mieux encore à température ambiante.

Un autre objet de la présente invention est de fournir une composition émulsifiante à usage cosmétique à spectre large, c'est-à-dire polyvalente du point de vue des produits finaux envisagés : de ce point de vue, la composition selon l'invention peut être utilisée dans des produits aussi divers que des lotions, crèmes, gels, laits .... En outre, ladite composition est avantageusement non irritante et non allergique pour la peau. Elle offre de plus l'avantage de ne pas dépendre du pH ou de la présence d'électrolytes : en d'autres termes, sa capacité émulsifiante n'est pas affectée par le pH du milieu, ni par la présence de sels mono, di ou trivalents. Ce critère est d'autant plus important que de manière générale, les produits à usage cosmétique et notamment pour application topique sont susceptibles d'être soumis ou exposés à des variations de pH (à titre d'exemple, le pH de la peau est légèrement acide, et varie entre 4 et 6). Disposer d'un produit qui ne présente pas de limite particulière d'usage en matière de pH représente donc un très grand avantage technique pour une composition cosmétique.

Enfin, un autre objet de la présente invention est de fournir une composition à usage cosmétique, offrant une large palette de propriétés sensorielles d'intérêt, tel que notamment une sensation de fraicheur au niveau de l'épiderme ou une texture onctueuse. En effet, la composition émulsifiante selon l'invention permet la réalisation aisée d'émulsions H/E à la fois très stables et très fines, avec des textures modulables et présentant un toucher frais, soyeux et non gras, même pour des teneurs élevées en phase grasse dispersée. Il est ainsi possible d'obtenir des émulsions ayant un bon effet émollient sur la peau ainsi qu'un bon effet hydratant des couches supérieures de l'épiderme.

L'ensemble de ces objectifs qui constitue un problème technique complexe à résoudre, est finalement atteint grâce au principal objet de la présente invention, qui consiste en une composition émulsifiante, notamment à usage cosmétique, apte à permettre l'obtention d'une émulsion de type huile dans eau (H/E), comprenant:

1) au moins une cyclodextrine,
2) et au moins un émulsifiant H/E d'origine naturelle.

l'émulsifiant H/E présentant une balance Hydrophile-Lipophile (HLB) supérieure à 8, préférentiellement supérieure ou égale à 9.

Le calcul de la HLB prend en compte les masses moléculaires des parties hydrophiles et la masse moléculaire de la molécule considérée et peut s'obtenir selon l'équation suivante :

$$HLB = 20 \, \frac{\text{Masse moléculaire partie hydrophile}}{\text{Masse moléculaire de la molécule}}$$

Dans la présente Demande, le terme « cyclodextrine » désigne et inclut l'une quelconque de cyclodextrines connues par ailleurs, telles que les cyclodextrines natives et non substituées contenant de 6 à 12 unités de glucose liées par des liaisons covalentes entre les carbones 1 et 4, et notamment les alpha-, beta- et gamma- cyclodextrines contenant respectivement 6, 7 et 8 unités de glucose.

Ce terme recouvre également des « dérivés de cyclodextrine » à savoir des molécules dont une partie au moins des groupements hydroxyles OH a été transformée en groupements OR, où R désigne de manière générale un groupement alkyl. De ce point de vue, les dérivés de cyclodextrine incluent notamment les cyclodextrines méthylées, éthylées, mais aussi celles substituées avec un groupement hydroxyalkyl telles que les cyclodextrines hydroxypropylées et hydroxyéthylées.

Les cyclodextrines préférées selon la présente invention sont les alpha-, beta- et gamma-cyclodextrines, la beta-cyclodextrine native étant la plus préférée.

La cyclodextrine pourra notamment se présenter sous forme d'une poudre cristalline, pseudo-cristalline ou amorphe.

Dans la présente Demande, le terme « émulsifiant H/E d'origine naturelle » désigne toute molécule issue de ressources renouvelables, notamment extraite ou secrétée par des plantes, des micro-organismes ou d'algues et apte à permettre après modification physique, chimique ou enzymatique, l'obtention d'une émulsion de type eau dans huile H/E ou à en favoriser la stabilité. Sous cette définition, sont donc inclus des produits appelés co-émulsifiants H/E d'origine naturelle.

La Demanderesse a pu constater après maints essais et plans d'expérience, que de façon tout à fait surprenante et inattendue, l'association à une cyclodextrine d'un émulsifiant d'origine naturelle présentant une balance Hydrophile-Lipophile (HLB) supérieure à 8, préférentiellement supérieure ou égale à 9, permettant de façon connue en soi d'obtenir une émulsion H/E, loin d'avoir des conséquences négatives sur la stabilité et la qualité d'émulsions H/E, a au contraire un effet très favorable. Il est ainsi avantageusement possible de réduire la quantité d'émulsifiant d'origine naturelle requis pour obtenir une émulsion satisfaisante et stable, et de plus, d'ajuster finement la texture et le profil sensoriel de l'émulsion.

Sans vouloir se lier à une quelconque théorie, il semble que la présence de très faibles quantités d'un émulsifiant H/E d'origine naturelle, facilite grandement la formation in situ de complexes d'inclusion entre la cyclodextrine et certaines molécules spécifiques présentes de la phase grasse dispersée, et cela sous forme de particules colloïdales, solides ou semi-liquides, qui se placent aux interfaces huile et eau. Ces particules sont très compatibles physiquement et sensoriellement avec la peau ou les cheveux et n'endommagent pas les membranes cellulaires de nature animale.

Il a été montré que l'association de cyclodextrine à de faibles quantités d'un émulsifiant H/E d'origine naturelle présentant une balance Hydrophile-Lipophile (HLB) supérieure à 8, préférentiellement supérieure ou égale à 9, permet d'obtenir des émulsions très stables avec des gouttes de taille inférieure à 30 $\mu$m, voire inférieure à 10$\mu$m. Il a été aussi montré que cet ajout permet d'ajuster la viscosité finale de l'émulsion H/E. Grâce à cet ajustement de la viscosité, combiné à la taille de gouttes inférieure à 30 $\mu$m, voire 10 $\mu$m, la texture des émulsions peut être avantageusement ajustée.

L'emulsifiant H/E d'origine naturelle ayant une balance Hydrophile-Lipophile (HLB) supérieure à 8, préférentiellement supérieure ou égale à 9, est de préférence présent dans la composition émulsifiante dans un ratio compris entre 0,01 et 1 part, de préférence compris entre 0,05 et 0,5 part, plus préférentiellement compris entre 0,10 et 0,35 part, et mieux encore compris entre 0,15 et 0,30 part, pour 1 part en poids de cyclodextrine.

Cet émulsifiant H/E d'origine naturelle peut être choisi également parmi les produits naturellement biodégradables en milieu naturel hydraté, avec notamment une balance Hydrophile-Lipophile (HLB) comprise entre 8 et 20, de préférence entre 9 et 16, et mieux encore entre 11 et 14.

A titre d'exemple, cet émulsifiant H/E d'origine naturelle peut être choisi parmi les produits suivants, pour peu qu'ils satisfassent à la condition sur le HLB ci-dessus : les alkyl polyglucosides ; les mélanges d'au moins un alkyl polyglucoside et d'au moins un alcool gras ; les esters gras non éthoxylés de polyols, et notamment parmi les esters gras non éthoxylés de glycérol, de polyglycérols, de sorbitol, de sorbitan, d'anydrodrohexitols comme en particulier l'isosorbide, de mannitol, de xylitol, d'érythritol, de maltitol, de saccharose, de glucose, de polydextrose, de sirops de glucose hydrogéné, de dextrines et d'amidons hydrolysés.

L'émulsifiant H/E d'origine naturelle est de préférence choisi pour être naturellement biodégradable en milieu naturel hydraté. Il peut s'agir notamment d'esters gras non éthoxylés de polyols obtenus à partir d'acide gras ou par trans-estérification à partir d'huile ou de mélanges d'huiles. Les acides gras utilisés comprennent de 8 à 22 atomes de carbone, de préférence de 10 à 18 atomes de carbone, et en particulier de 12 à 18 atomes de carbone. Ces acides peuvent être linéaires ou ramifiés, saturés ou insaturés, posséder une ou des fonctions hydroxyles latérales. Les huiles peuvent être saturées ou insaturées, de liquide à solide à température ambiante, et posséder éventuellement des fonctions hydroxyle, de préférence d'indice d'iode compris entre 1 et 145, et en particulier de 5 à 105.

L'émulsifiant H/E d'origine naturelle peut être en particulier choisi parmi les esters de polyglycérols, et de préférence parmi les esters issus de la réaction de polyglycérols comprenant de 2 à 12 unités de glycérol, de préférence de 3 à 10 unités glycérol avec au moins une huile végétale partiellement hydrogénée ou non hydrogénée d'indice d'iode compris entre 1 et 15, et en particulier de 5 à 10. Il peut s'agir en particulier des esters oléiques, stéariques, palmitiques, lauriques, diisostéariques et capryliques de polyglycérols et en particulier des produits suivants : Polyglyceryl-5 Dioleate de HLB environ 8 (comme Dermofeel® G 5 DO de Evonik Dr. Straetmans GmbH), Polyglyceryl-2 Caprate de HLB environ 9 (comme HYDRIOL® PGC.2 de HYDRIOR), Polyglyceryl-3 Stéarate de HLB environ 9 (comme Dermofeel® PS de Evonik Dr. Straetmans GmbH), Polyglyceryl-2 Laurate de HLB environ 9 (comme Dermofeel® G2L de Evonik Dr. Straetmans GmbH), Polyglyceryl-3 Palmitate de HLB environ 10 (comme Dermofeel® PP de Evonik Dr. Straetmans GmbH), Polyglyceryl-10 Diisostearate de HLB environ 11 (comme Dermofeel® G10 DI de Evonik Dr. Straetmans GmbH), Polyglycéryl-6 Caprylate de HLB environ 11,5, Polyglycéryl-5 Laurate de HLB environ 13 (comme Dermofeel® G5L de Evonik Dr. Straetmans GmbH), Polyglyceryl-3 Caprate de HLB environ 14 (comme HYDRIOL® PGC.3 de HYDRIOR), Polyglyceryl-4 Caprate de HLB environ 14 (comme MASSOCARE PG4 C de Masso), Polyglyceryl-10 Monolaurate de HLB environ 14,8 , Polyglyceryl-6 Caprylate de HLB environ 15 (comme Dermofeel® G 6 CY de Dr. Straetmans GmbH / Evonik), Polyglyceryl-10 Laurate de HLB environ 16 (comme Dermofeel® G 10 L de Dr. Straetmans GmbH / Evonik).

L'émulsifiant H/E d'origine naturelle est de préférence choisi parmi les alkyl polyglucosides, parfois aussi appelé alkyl polyglycosides, et désigné par l'acronyme APG. Ces émulsifiants sont des tensioactifs non-ioniques bien connus en soi. Le brevet FR 2 948 285 en fait une présentation en termes de structure, et explique comment les préparer. Ils peuvent être représentés par la formule générale (I) suivante : $R_1\text{-O-}(R_2\text{-O})_p\text{-}(S)_n$

Dans laquelle :

- S représente un saccharide réducteur, pouvant comprendre entre 5 et 6 atomes de carbone,

- R1 désigne un radical alkyl et/ou alcényle linéaire ou ramifié comportant environ 8 à 24 atomes de carbones, ou un radical alkylphényle dont le groupe alkyle linéaire ou ramifié comporte environ de 8 à 24 atomes de carbone,

- R2 désigne un radical alkylène comportant de 2 à 4 atomes de carbone,

- n désigne une valeur allant de 1 à 15,

- p désigne une valeur allant de 0 à 10.

Par saccharide réducteur, on désigne dans la formule (I), les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990. La structure oligomérique $(S)_n$, peut se présenter sous toute forme d'isomérie, qu'il s'agisse d'isomérie optique, d'isomérie géométrique ou d'isomérie de position ; elle peut aussi représenter un mélange d'isomères.

Selon un aspect particulier de la présente invention, dans la définition des composés de formules (I), S représente un saccharide réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose et plus particulièrement un saccharide réducteur choisi parmi le glucose, le xylose ou l'arabinose.

Une première variante préférée d'alkyl polyglucosides selon la présente invention sont les alkyl glucosides en C12-C20, c'est-à-dire les composés de formule (I) dans laquelle :

- R1 désigne plus particulièrement un radical alkyle et/ou alcényle linéaire ou ramifié comportant environ de 12 à 20 atomes de carbones

- p prend une valeur allant de 0 à 3, et préférentiellement égale à zéro,

- S désigne le glucose, le fructose ou le galactose, et plus préférentiellement le glucose

Une seconde variante préférée d'alkyl polyglucosides selon la présente invention sont les alkyl glucosides en C12-C20 de la première variante préférée dans lesquels :

- R1 désigne plus particulièrement un radical alkyle linéaire comportant environ de 12 à 20 atomes de carbones

- p est égal à zéro,

- S désigne le glucose

Des alkyl polyglucosides de formule (I) sont notamment disponibles dans le commerce sous les noms : Plantacare® 810 UP ($R_1$ est en C8-C10 / INCI : caprylyl/capryl glucoside), Plantacare® 818 UP ($R_1$ est en C8-C16 / INCI : Coco-glucoside), Plantacare® 2000 UP ($R_1$ est en C8-C16 / INCI : decyl glucoside) et Plantacare® 1200 UP ($R_1$ est en C12-C16 / INCI : lauryl glucoside) vendus par la société BASF ; Macanol® 810 ($R_1$ est en C8-C10), Macanol® 1200 ($R_1$ est en C12-C14), Macanol® 816 (mélange de $R_1$ est en C8, C10, C12, C14, C16) vendus par la société FCI Technology; Neocare MF 0718 ($R_1$ est en C8-C10 / INCI : caprylyl/capryl glucoside), Neocare MF 0012 ($R_1$ est en C12-C14 / INCI :

lauryl glucoside), Neocare MF 0002 ($R_1$ est en C8-C16 / INCI : decyl glucoside), Neocare MF 818 ($R_1$ est en C8-C16 / INCI : coco glucoside) vendus par la société Neochem ; Tego Care CG 90 ($R_1$ est en C14-C16 / INCI : cetearyl glucoside) vendus par la société Evonik Healthcare.

L'émulsifiant H/E d'origine naturelle peut être un mélange constitué d'au moins un alkyl polyglucoside et d'au moins un alcool gras. Dans ces mélanges, les alkyl polyglucosides peuvent être choisis parmi tous les alkyl polyglucosides utiles à l'invention décrits précédemment. Concernant les alcools gras utiles au mélange avec les alkyl glucosides, on trouvera les alcools gras linéaires ou ramifiés ayant un nombre total d'atome de carbone allant de 8 à 24.

Des mélanges d'alkyl polyglucosides et d'alcools gras utiles à l'invention et disponibles dans le commerce sont ceux vendus par la société SEPPIC : Montanov™ 14 (INCI : Myristyl Alcohol & Myristyl Glucoside), Montanov™ 202 (INCI : Arachidyl Alcohol and Behenyl Alcohol and Arachidyl Glucoside), Montanov™ 68 (INCI : Cetearyl Alcohol & Cetearyl Glucoside), Montanov™ 82 (INCI : Cetearyl Alcohol and Coco-Glucoside), Montanov™ S (INCI : Coco-Glucoside & Coconut Alcohol), Montanov™ L (INCI : C14-22 Alcohols & C12-20 Alkyl Glucoside).

Le mélange d'alkyl polyglucoside et d'alcool gras préféré est celui commercialisé par SEPPIC sous le nom « Montanov™ L », qui est un mélange d'alcools gras en C14-C22 et d'alkyl polyglucosides en C12-C20 (INCI : C14-22 Alcohols & C12-20 Alkyl Glucoside). Le mélange d'alkyl polyglucoside et d'alcool gras plus préféré est celui commercialisé par SEPPIC sous le nom « Montanov™ 68» (INCI : Cetearyl Alcohol & Cetearyl Glucoside).

L'émulsifiant H/E d'origine naturelle peut être également choisi parmi les esters gras de dextrines ou d'amidons hydrolysés, notamment sous forme d'esters octénylsuccinate de sodium. Il pourra s'agir par exemple des produits commercialisés par la Demanderesse sous les noms ce CLEARGUM® et notamment les produits CLEARGUM® CO 01 et CLEARGUM® CO 03.

La composition émulsifiante selon l'invention permet d'obtenir des effets sensoriels intéressants, comme une texture particulière ou une sensation de fraicheur, selon les proportions utilisées.

Les propriétés intéressantes de ladite composition émulsifiante résultent de la combinaison des deux composés utilisés qui présentent une bonne synergie à la fois en termes de stabilité des émulsions que de propriétés sensorielles. Bien que l'on obtienne des résultats très satisfaisants quelles que soient les proportions dans lesquelles on associe ces composés, on obtient des résultats particulièrement probants lorsqu'ils sont associés dans un ratio très précis.

En particulier, chacun des différents constituants de la présente composition émulsifiante selon l'invention peut être intégré à une phase différente, avant qu'il soit procédé à la mise en émulsion. Alternativement, les différents composés de la présente composition selon l'invention sont mélangés entre eux pour constituer ce qui sera désigné dans la suite par le terme « système émulsifiant », ledit système émulsifiant pouvant être ajouté à l'une ou l'autre des deux phases non miscibles pour permettre la formation d'une émulsion H/E. L'invention permet avantageusement ces deux modes de réalisation avec les mêmes composés, ce qui permet une marge de manœuvre accrue et une utilisation simplifiée.

Une telle composition émulsifiante selon l'invention présente notamment l'avantage d'être complètement d'origine naturelle, et d'être utilisable selon un procédé à froid et en particulier à température ambiante. Ladite composition selon l'invention est à usage cosmétique et, à ce titre, n'est pas sensible à des variations de pH raisonnables ou de salinité du milieu, n'est pas irritante et n'est pas susceptible de provoquer des allergies, notamment cutanées. En outre, la composition selon l'invention peut être utilisée pour réaliser tout type d'émulsion, notamment des émulsions de type Pickering, et convient donc à une grande variété d'usages : crèmes, laits, sérums, lotions, etc.

La composition émulsifiante selon l'invention permet de réaliser des émulsions de type Pickering pouvant avantageusement être stabilisées par des particules organiques compatibles avec la peau ou le cheveu. Elle peut comprendre de façon complémentaire d'autres produits aptes à former ou à stabiliser des émulsions de Pickering comme des silices et des amidons granulaires octénylsuccinates sous forme de sels de calcium ou d'aluminium.

De préférence, la composition émulsifiante comprend également au moins un polyol.

Les polyols visés par la présente Demande sont tous les polyols connus par ailleurs, et notamment le maltitol, le mannitol, le xylitol, l'érythritol, le sorbitol, le glycérol, le glycérol et le sorbitol étant les polyols préférés. De préférence, ce polyol est cristallisé ou bien se présente sous la forme d'une poudre.

Selon la présente invention, la composition émulsifiante, notamment à usage cosmétique et apte à permettre l'obtention d'une émulsion de type huile dans eau (H/E), contient, en % en poids :

1) de 40 % à 95 % d'au moins une cyclodextrine,
2) de 5 % à 40 % d'au moins un émulsifiant H/E d'origine naturelle, de préférence choisi parmi les alkyl polyglucosides, les mélanges d'au moins un alkyl polyglucoside et d'au moins un alcool gras, et les esters gras non éthoxylés de polyols, ayant une balance Hydrophile-Lipophile (HLB) supérieure à 8, préférentiellement supérieure ou égale à 9,
3) et de 0% à 40 % d'au moins un polyol.

De manière préférée, cette composition émulsifiante selon la présente invention est caractérisée en ce qu'elle contient, en % en poids :

1) de 45 % à 85 % d'au moins une cyclodextrine,
2) de 5 % à 30 % d'au moins un émulsifiant H/E d'origine naturelle, de préférence choisi parmi les alkyl polyglucosides, les mélanges d'au moins un alkyl polyglucoside et d'au moins un alcool gras, et les esters gras non éthoxylés de polyols, ayant une balance Hydrophile-Lipophile (HLB) supérieure à 8, préférentiellement supérieure ou égale à 9,
3) et de 10 % à 40 % d'au moins un polyol.

De la manière la plus préférée, cette composition selon la présente invention est caractérisée en ce qu'elle contient, en % en poids :

1) de 40 % à 80 % d'au moins une cyclodextrine,
2) de 10 % à 20 % d'au moins un émulsifiant H/E d'origine naturelle, de préférence choisi parmi les alkyl polyglucosides, les mélanges d'au moins un alkyl polyglucoside et d'au moins un alcool gras, et les esters gras non éthoxylés de polyols, ayant une balance Hydrophile-Lipophile (HLB) supérieure à 8, préférentiellement supérieure ou égale à 9,
3) et de 10% à 30 % d'au moins un polyol.

De préférence l'émulsifiant H/E d'origine naturelle ayant une balance Hydrophile-Lipophile (HLB) supérieure à 8 est un produit naturellement biodégradable en milieu naturel hydraté, et tout préférentiellement un mélange d'au moins un alkyl polyglucoside et d'au moins un alcool gras.

Un autre objet de la présente invention a trait à une composition de type émulsion de Pickering H/E, notamment à usage cosmétique, caractérisée en ce qu'elle contient pour 1 part en poids d'au moins une cyclodextrine, entre 0,01 et 1 part, de préférence entre 0,05 et 0,5 part, plus préférentiellement entre 0,10 et 0,35 part, et mieux encore entre 0,15 et 0,30 part d'au moins un émulsifiant H/E d'origine naturelle, ce dernier étant de préférence choisi parmi les alkyl polyglucosides, les mélanges d'au moins un alkyl polyglucoside et d'au moins un alcool gras, et les esters gras non éthoxylés de polyols, ayant une balance Hydrophile-Lipophile (HLB) supérieure à 8, préférentiellement supérieure ou égale à 9.

La composition de type émulsion de Pickering H/E selon l'invention peut comprendre en outre une phase grasse qui peut être à température ambiante (25°C), liquide comme par exemple les huiles végétales, ou bien solide comme dans le cas de cires. Cette phase grasse liquide peut être d'origine minérale, animale, végétale ou de synthèse et être composée d'huiles hydrocarbonées voir éventuellement des huiles de silicones. Par huile hydrocarbonée, on entend une huile formée essentiellement, voire constituée, d'atomes de carbone et d'hydrogène et éventuellement d'atome d'oxygène, d'azote, pouvant contenir des groupes alcool, ester, éther, acide carboxylique, amine et/ou amide.

De préférence, cette composition contient une ou plusieurs huiles liquides à température ambiante (25°C), de préférence au moins une huile liquide non volatile. On entend par huile liquide non volatile, une huile susceptible de rester sur la peau à température ambiante, à pression atmosphérique pendant au moins une heure.

La phase grasse liquide comprend avantageusement une ou plusieurs huiles non volatiles qui procurent un effet émollient sur la peau. On peut citer les esters gras tels que l'isononoate de cetearyl, l'isononoate d'isotridecyl, l'isostearate d'isostearyl, l'isostearate d'isopropyl, le myristate d'isopropyl, le palmitate d'isopropyl, stéarate de butyle, le laurate d'hexyle, l'isononate d'isononyle, le palmitate de 2-éthyl-hexyle, le laurate de 2-hexyldécyle, le palmitate de 2-octyl décyle, le myristate ou la lactate de 2-octyldodécyle, le succinate de 2-diéthyl hexyle, le malate de diisostéaryle, la tracétine , la tricprine , les triglycerides d'acide caprylique/caprique, le triisostéarate de glycérine, l'acetate de tocopherol, les acides gras supérieurs tels que l'acide myristique, l'acide palmitique, l'acide stéarique, l'acide béhénique, l'acide oléique, l'acide linoléique, l'acide linolénique ou l'acide isostéarique, , les alcools gras supérieurs tel que l'alcool oléique, les huiles végétales comme l'huile d'avocat, l'huile de camélias, l'huile de noisette, l'huile de tsubaki, l'huile de noix de cajou, l'huile d'argan, l'huile de soja, l'huile de pépins de raisin, l'huile de sésame, l'huile de mals, l'huile de germes de blé, l'huile de colza, l'huile de tournesol, l'huile de coton, l'huile de jojoba, l'huile d'arachide, l'huile d'olive et leurs mélanges, les beurres végétaux comme le beurre de karité, le beurre de camellia.

Ces huiles peuvent être des huiles de type hydrocarbures ou siliconées telles que l'huile de paraffine, de squalane, la vaseline, les diméthyls siloxanes et leurs mélanges.

La phase grasse liquide peut aussi comprendre éventuellement des huiles volatiles. Par huile volatile, on entend une huile susceptible de s'évaporer de la peau, en moins d'une heure à température ambiante et pression atmosphérique. Les huiles volatiles peuvent être par exemple choisies parmi les huiles de silicones ou triglycérides d'acides gras courts pour réduire le toucher gras.

De préférence la composition de type émulsion de Pickering H/E selon l'invention ne contient que des huiles d'origine renouvelable et notamment des huiles ou beurres d'origine végétale, de préférence raffinés. Ces huiles et beurres s'accordent parfaitement bien au système émulsifiant objet de l'invention au sens où elles permettent d'obtenir des émulsions très stables, avec une blancheur élevée et une viscosité facilement ajustable.

La composition émulsifiante selon l'invention permet avantageusement de préparer des émulsions H/E à très hautes teneurs en huile. Ce type d'émulsions H/E riche en huile est d'ordinaire difficile à obtenir sous forme stable dans le temps avec des émulsifiants classiques. La teneur en huile de l'émulsion H/E finale est de préférence comprise entre 10 et 65 % en poids, et de préférence de l'ordre de 20 à 55 % en poids. Les huiles végétales ou d'origine végétale comme par exemple l'huile de tournesol et l'isopropyl palmitate permettent en particulier d'obtenir des émulsions stables, ne donnant pas lieu à un crémage ou à un déphasage.

La composition selon l'invention peut comprendre en outre un agent de rhéologie comme notamment un agent épaississant de la phase aqueuse, un agent gélifiant ou un agent suspensif, tels que par exemple les gommes issues de plantes comme la gomme arabique, la gomme de konjac , la gomme de guar ou leurs dérivés ; les gommes extraits d'algues comme les alginates ou les carraghénanes ; les gommes issues d'une fermentation microbienne comme les xanthanes, les mannanes , les scléroglucanes ou leurs dérivés ; la cellulose et ses dérivés comme la carboxyméthylcellulose ou l'hydroxyéthylcellulose ; l'amidon et ses dérivés comme en particulier les amidons modifiées, notamment acétylés, carboxyméthylés, octénylsuccinates ou hydroxypropylés ; les polymères synthétiques comme les polyacide-acryliques ou les carbomères.

De préférence la composition selon l'invention comprend un agent de rhéologie choisi parmi les polysaccharides naturels issus de plantes ou de fermentation, éventuellement modifiés. La xanthane et ses dérivés permettent en particulier d'obtenir des émulsions H/E avec des tailles de gouttelettes très fines, même utilisés à une teneur inférieure à 1% en poids de l'émulsion totale.

Les émulsions obtenues par emploi de la composition émulsifiante selon l'invention présentent de préférence une taille de goutte inférieure ou égale à 30 $\mu$m, préférentiellement inférieure ou égale à 10 $\mu$m. Par taille de goutte de l'émulsion, on entend le diamètre moyen des gouttes de phase grasse dispersée en suspension dans la phase aqueuse. Une faible taille de goutte augmente la stabilité de l'émulsion en réduisant la vitesse de floculation de l'émulsion, et donc la vitesse de la séparation de phase. La taille de goutte dépend d'un grand nombre de paramètres et, à ce titre, constitue une caractéristique qu'il convient de contrôler et qui n'est pas intrinsèque à la formulation du système émulsifiant. La taille des gouttelettes est mesurée au moyen d'un microscope optique LEICA DMLS au grossissement $\times$10.

La composition selon l'invention peut comprendre en outre un conservateur choisi parmi l'alcool benzylique, l'acide déhydroacétique et leurs mélanges.

La composition selon l'invention présente de préférence une viscosité Brookfield supérieure à 3 000 mPas à 25°C, de préférence supérieure à 5 000 mPas à 25°C. La viscosité est mesurée à l'aide d'un viscosimètre Brookfield DV-II+Pro mis en rotation à une vitesse de 20 rotations par minute au contact de l'échantillon produit. La résistance du produit à ce mouvement de rotation est enregistrée pendant une minute et convertie en mPascal.seconde. Pour chaque échantillon, la viscosité est mesurée trois fois et la moyenne arithmétique des trois valeurs est retenue. Une broche adaptée à la viscosité mesurée est sélectionnée selon les gammes suivantes : la broche choisie est la broche SP3 quand la viscosité est inférieure ou égale à 5 000 mPa.s, SP4 quand la viscosité est comprise entre 5 000 mPa.s et 7 000 mPa.s et SP5 quand la viscosité est supérieure ou égale à 7 000 mPa.s.

Afin de caractériser les propriétés sensorielles des systèmes émulsifiants selon l'invention, on utilise des descripteurs sensoriels et un protocole d'évaluation sensoriel correspondant en 5 étapes. Ces 5 étapes correspondent aux différentes phases d'application d'un produit de soin : l'aspect, la prise en main, l'étalement après 1 minute, après 2 minutes. Durant ces 5 phases, plusieurs descripteurs sensoriels sont évalués par un panel d'évaluateurs qui attribuent un score pouvant aller de 0 à 10.

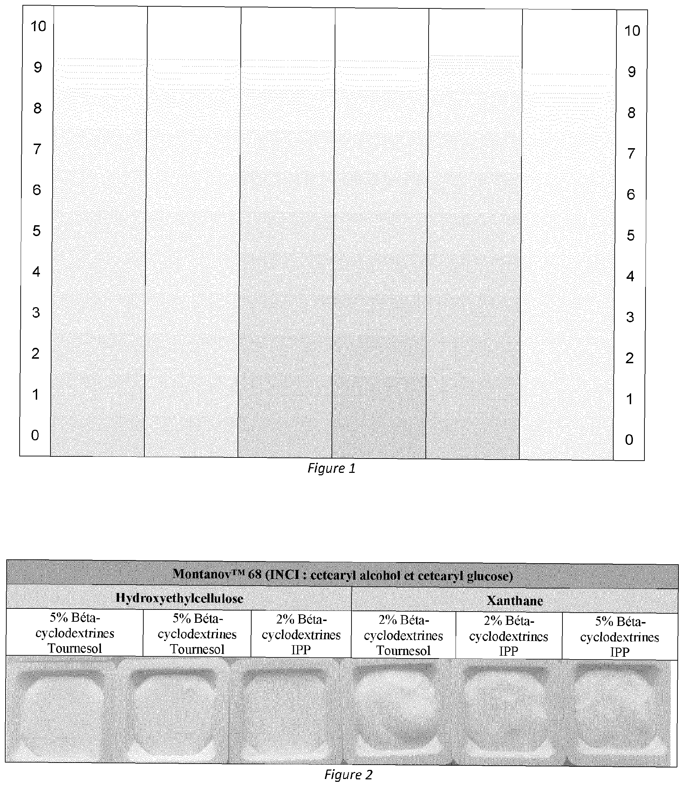

La composition selon l'invention présente de préférence un descripteur de blancheur supérieur à 8. Le descripteur de blancheur est défini par la palette de couleurs de la figure 1. Le produit est examiné sous la lampe et comparé à la palette de couleur par un panel d'évaluateurs.

La composition selon l'invention présente de préférence un descripteur de brillance supérieur ou égal à 8. La brillance est définie par la propension du produit à réfléchir la lumière.

La composition selon l'invention présente de préférence un descripteur d'étalement supérieur à 8. L'étalement est évalué en examinant le produit après avoir déposé sur la main 50 à 100$\mu$l du produit, pendant son étalement en 10 rotations, sous une lampe. L'étalement est d'autant plus important qu'il y a peu de résistance au mouvement entre le 5e et le 10e tour sur la main.

La composition selon l'invention présente de préférence un descripteur filmogène supérieur à 8. Le descripteur filmogène correspond à la propension du produit à former un film continu en effectuant un glissement sur la peau 2 minutes après avoir effectué 10 rotations.

Un autre objet de la présente invention consiste en un procédé de fabrication d'une composition de type émulsion de Pickering H/E, notamment à usage cosmétique, comprenant les étapes suivantes :

a) la dispersion dans une phase aqueuse d'une composition émulsifiante comprenant au moins une cyclodextrine et au moins un émulsifiant H/E d'origine naturelle, ce dernier étant présent dans un ratio compris entre 0,01 et 1 part, de préférence entre 0,05 et 0,5 part, plus préférentiellement entre 0,10 et 0,35 part, et mieux encore entre 0,15 et 0,30 part, pour 1 part en poids d'au moins cette cyclodextrine ; cet émulsifiant H/E d'origine naturelle pouvant être choisi en particulier parmi au moins un alkyl polyglucoside, au moins un mélange d'au moins un alkyl polyglucoside et d'au moins un alcool gras, et au moins un ester gras non éthoxylé de polyols, plus préférentiellement choisi parmi les mélanges d'au moins un alkyl polyglucoside et d'au moins un alcool gras, ayant une balance Hydrophile-Lipophile (HLB) inférieur à 8, préférentiellement supérieure ou égale à 9,

b) l'ajout au mélange obtenu à l'étape a) d'une phase grasse, de préférence en une quantité comprise entre 10 et 65 % en poids, et de préférence de l'ordre de 20 à 55 % en poids par rapport au poids final de l'émulsion, sous une agitation mécanique suffisante pour permettre la dispersion de la phase grasse en fines gouttelettes dans la phase aqueuse et l'obtention d'une émulsion H/E ayant une taille de goutte inférieure ou égale à 30 μm, préférentiellement inférieure ou égale à 10 μm.

Selon une variante, le procédé selon l'invention de fabrication d'une composition de type émulsion de Pickering H/E, notamment à usage cosmétique, comprenant les étapes suivantes :

a) la dispersion dans une phase grasse d'une composition émulsifiante comprenant au moins une cyclodextrine et au moins un émulsifiant H/E d'origine naturelle, ce dernier étant présent dans un ratio compris entre 0,01 et 1 part, de préférence entre 0,05 et 0,5 part, plus préférentiellement entre 0,10 et 0,35 part et mieux encore entre 0,15 et 0,30 part pour 1 part en poids d'au moins cette cyclodextrine ; cet émulsifiant H/E d'origine naturelle pouvant être choisi en particulier parmi au moins un alkyl polyglucoside, un mélanges d'au moins un alkyl polyglucoside et d'au moins un alcool gras, et un ester gras non éthoxylé de polyols, plus préférentiellement choisi parmi les mélanges d'au moins un alkyl polyglucoside et d'au moins un alcool gras, ayant une balance Hydrophile-Lipophile (HLB) supérieure à 8, préférentiellement supérieure ou égale à 9; la phase grasse représentant de préférence entre 10 et 65 % en poids, et de préférence de l'ordre de 20 à 55 % en poids du poids final de l'émulsion,

b) et l'ajout du mélange obtenu à l'étape a) à une phase aqueuse_sous une agitation mécanique suffisante pour permettre la dispersion de la phase grasse en fines gouttelettes dans la phase aqueuse et l'obtention d'une émulsion H/E ayant une taille de goutte inférieure ou égale à 30 μm, préférentiellement inférieure ou égale à 10 μm.

L'invention pourra être mieux comprise à l'aide des exemples de réalisation non limitatifs décrits ci-après.

**EXEMPLE 1**

L'invention pourra être mieux comprise à l'aide des exemples de réalisation non limitatifs décrits ci-après, et à l'examen des figures annexées :

- la figure 1 représente la palette de couleur utilisée pour l'évaluation du descripteur de blancheur ;

- la figure 2 est une photographie de 6 émulsions H/E obtenues à partir de compositions émulsifiantes selon l'invention.

On réalise 6 compositions sous forme d'émulsion H/E selon l'invention en utilisant le protocole suivant. Les compositions de ces 6 compositions sont reportées dans le tableau 1.

On commence par disperser un agent gélifiant dans de l'eau sous agitation avec une pâle à défloculation à 1000 tours par minute. La température de l'eau est fixée à 40°C lorsque l'agent gélifiant est de la gomme de xanthane, et à 70°C lorsque l'agent gélifiant est de l'hydroxyethylcellulose.

On mouille alors la béta-cyclodextrine dans de la glycérine et on ajoute le mélange béta-cyclodextrine/glycérine au mélange eau/agent gélifiant sous agitation 1000 tours par minute, afin d'obtenir une phase aqueuse.

La quantité de béta cyclodextrines est fixée à 2% ou à 5% en masse de la composition selon les cas.

A part, on ajoute l'alkyl polyglucoside MONTANOV 68 (INCI : cetearyl alcohol & cetearyl glucose), de la société SEPPIC, dans de l'huile de tournesol ou dans de l'isopropyl palmitate sous agitation magnétique, à 40°C pour obtenir une phase huileuse.

La phase huileuse est alors émulsionnée dans la phase aqueuse à 40°C sous agitation à 1500 tours par minute, pendant 15 minutes.

On ajoute un conservateur (mélange à base d'alcool benzylique et d'acide déhydroacétique).

*Tableau 1*

| | % Béta-cyclodextrine | Huile (30%) | Emulsionnant (1%) | Epaississant (0,7%) |
|---|---|---|---|---|
| 1 | 5 | Tournesol | Montanov™ 68 | Hydroxyethyl Cellulose |
| 2 | 5 | Tournesol | Montanov™ 68 | Hydroxyethyl Cellulose |
| 3 | 2 | Isopropyl Palmitate (IPP) | Montanov™ 68 | Hydroxyethyl Cellulose |

(suite)

|   | % Béta-cyclodextrine | Huile (30%) | Emulsionnant (1%) | Epaississant (0,7%) |
|---|---|---|---|---|
| 4 | 2 | Tournesol | Montanov™ 68 | Xanthane |
| 5 | 2 | Isopropyl Palmitate (IPP) | Montanov™ 68 | Xanthane |
| 6 | 5 | Isopropyl Palmitate (IPP) | Montanov™ 68 | Xanthane |

La figure 2 est une photographie des 6 émulsions H/E obtenus selon les formulations données dans le tableau 1.

On constate que toutes les émulsions selon l'invention comprenant 1% de Montanov™ 68 (cetearyl alcohol et cetearyl glucose) et 2 ou 5 % de béta-cyclodextrine permettent d'obtenir des émulsions H/E stables et cela en utilisant seulement 1 %m d'émulsifiant H/E habituel.

Pour chacune de ces compositions, on mesure la viscosité, la taille des gouttes, la blancheur, l'étalement, le caractère gras, et le caractère pénétrant. Les compositions pourront être désignées indifféremment par les termes *échantillons* ou *produit* dans la suite de la présente description détaillée.

La viscosité est mesurée à l'aide d'un viscosimètre Brookfield DV-II+Pro. Un mobile de taille fixe (mobiles SP2 à SP7 utilisés selon les niveaux de viscosités conformément aux consignes de l'appareil) est mis en rotation à une vitesse de 20 tours par minute au contact de l'échantillon produit. La résistance du produit à ce mouvement de rotation est enregistrée pendant une minute et convertie en milliPascal.seconde, noté mPa.s. Pour chaque échantillon, la viscosité est mesurée trois fois et la moyenne arithmétique des trois valeurs est retenue.

Le descripteur de blancheur est défini par la palette de couleurs de la figure 1. Le produit est examiné sous la lampe et comparé à la palette de couleur par un panel d'évaluateurs.

L'étalement est évalué en examinant le produit après avoir déposé sur la main 50 à 100 $\mu$l du produit, pendant son étalement en 10 rotations, sous une lampe. L'étalement est d'autant plus important qu'il y a peu de résistance au mouvement entre le 5$^e$ et le 10$^e$ tour sur la main.

Le caractère gras des compositions est évalué par un examen sous la lampe du produit, 1 minute après avoir effectué 10 rotations du produit sur la peau. Un panel d'évaluateur évalue alors la résistance du produit lorsque la peau sur laquelle il a été appliqué est placée entre le pouce et l'index et qu'un mouvement de frottement est appliqué. Le panel d'évaluateur prend également en compte l'aspect huileux ou non donné à la peau.

Enfin, le caractère pénétrant du produit est évalué par un examen sous la lampe du produit deux minutes après avoir effectué 10 rotations du produit sur la peau, en effectuant un glissement sur la peau. Un panel d'évaluateurs évalue alors la quantité de résidu de produit récupéré.

Les résultats obtenus pour les 6 compositions de type émulsion H/E sont consignés dans le tableau 2.

*Tableau 2*

|   | Viscosité en mPa.s (broche utilisée) | Taille des gouttes (microns) | Blanc | Etalement | Gras | Pénétrant |
|---|---|---|---|---|---|---|
| 1 | 12 120 (SP5) | Inférieure à 10 | 10 | 7 | 7 | 10 |
| 2 | 12 233 (SP5) | Inférieure à 10 | 10 | 7 | 7 | 10 |
| 3 | 4417 (SP3) | Inférieure à 10 | 10 | 8 | 5 | 10 |
| 4 | 4917 (SP3) | De 10 à 30 | 9 | 10 | 7 | 8 |
| 5 | 3940 (SP3) | De 10 à 20 | 10 | 10 | 6 | 10 |
| 6 | 4517 (SP3) | De 10 à 30 | 9 | 10 | 9 | 7 |

La viscosité des émulsions augmente légèrement quand la concentration en béta-cyclodextrine augmente : elle passe de 3 900 - 4 900 mPa.s avec 2% de béta-cyclodextrine à plus de 12 000 mPa.s avec 5%. De même, la viscosité est plus élevée lorsque l'épaississant utilisé est la cellulose au lieu de xanthane : elle passe de 4500 à 12 000 mPa.s. Les compositions émulsifiantes selon l'invention permettent avantageusement d'obtenir une très large gamme de viscosité, pouvant permettre de préparer aussi bien des laits que des crèmes visqueuses.

On peut noter que toutes les émulsions H/E obtenues en utilisant les compositions émulsifiantes selon l'invention, présentent d'excellentes propriétés sensorielles (notes allant de 6 à 10 selon les critères blanc, étalement, gras et pénétrant).

Les émulsions sont moins blanches lorsque l'huile utilisée est de l'huile de tournesol que lorsque l'huile utilisée est l'isopropyl palmitate (noté IPP) : le descripteur est de 10 sur 10 dans le cas de l'huile de tournesol, contre 9,33 sur 10 en moyenne dans le cas de l'isopropyl palmitate. Toutefois, la blancheur de l'isopropyl palmitate reste satisfaisante.

L'étalement est significativement plus facile avec le xanthane qu'avec la cellulose, il passe de 7,33 avec la cellulose à 10 avec le xanthane.

La présente description porte notamment sur les objets suivants :

1. Composition émulsifiante, notamment à usage cosmétique, apte à permettre l'obtention d'une émulsion de type huile dans eau (H/E), comprenant:

    a. au moins une cyclodextrine,
    b. et au moins un émulsifiant H/E, dans laquelle l'émulsifiant H/E est un mélange d'au moins un alkyl polyglucoside en C12-C20 et d'au moins un alcool gras linéaire ou ramifié ayant un nombre total d'atome de carbone allant de 8 à 24,
    l'émulsifiant H/E présentant une balance Hydrophile-Lipophile (HLB) supérieure à 8, préférentiellement supérieure ou égale à 9.

2. Composition émulsifiante selon la revendication 1, caractérisée en ce que l'émulsifiant H/E est présent dans la composition émulsifiante dans un ratio compris entre 0,01 et 1 part, de préférence compris entre 0,05 et 0,5 part, plus préférentiellement compris entre 0,10 et 0,35 part, et mieux encore compris entre 0,15 et 0,30 part, pour 1 part en poids de cyclodextrine.

3. Composition émulsifiante selon la revendication 1 ou 2, dans laquelle ladite au moins une cyclodextrine est choisie parmi les alpha-, beta- et gamma- cyclodextrines, de préférence parmi la beta-cyclodextrine native.

4. Composition émulsifiante selon l'une quelconque des revendications précédentes, dans laquelle la cyclodextrine se présente sous forme d'une poudre cristalline, pseudo-cristalline ou amorphe.

5. Composition émulsifiante selon l'une quelconque des revendications précédentes, dans laquelle l'émulsifiant H/E est choisi également parmi les produits naturellement biodégradables en milieu naturel hydraté avec une balance Hydrophile-Lipophile (HLB) comprise entre 8 et 20, de préférence entre 9 et 16 et mieux encore entre 11 et 14.

6. Composition émulsifiante selon l'une quelconque des revendications précédentes, dans laquelle l'émulsifiant H/E est un mélange constitué d'alcool gras en C14-C22 et d'alkyl polyglucoside en C12-C20.

7. Composition émulsifiante selon l'une quelconque des revendications 1 à 6 comprenant en outre au moins un polyol ; de préférence choisi parmi le maltitol, le mannitol, le xylitol, l'érythritol, le sorbitol, le glycérol.

8. Composition émulsifiante selon l'une quelconque des revendications 1 à 7 dans laquelle la composition contient, en % en poids :

    a. 40 % à 95 % d'au moins une cyclodextrine,
    b. 5 % à 40 % d' un mélange d'au moins un alkyl polyglucoside en C12-C20 et d'au moins un alcool gras linéaire ou ramifié ayant un nombre total d'atome de carbone allant de 8 à 24, ayant une balance Hydrophile-Lipophile (HLB) supérieure à 8, préférentiellement supérieure ou égale à 9,
    c. Et 0% à 40 % d'au moins un polyol.

9. Composition de type émulsion de Pickering H/E, notamment à usage cosmétique, caractérisée en ce qu'elle contient pour 1 part en poids d'au moins une cyclodextrine, entre 0,01 et 1 part, de préférence entre 0,15 et 0,30 part d' un mélange d'au moins un alkyl polyglucoside en C12-C20 et d'au moins un alcool gras linéaire ou ramifié ayant un nombre total d'atome de carbone allant de 8 à 24, ayant une balance Hydrophile-Lipophile (HLB) supérieure à 8, préférentiellement supérieure ou égale à 9.

10. Composition selon la revendication 9 contenant une ou plusieurs huiles liquides à température ambiante (25°C), de préférence au moins une huile liquide non volatile.

11. Composition selon la revendication 10, dans laquelle la teneur en huile de l'émulsion H/E finale est comprise entre 10 et 65 % en poids, et de préférence entre 20 et 55 % en poids.

12. Composition selon l'une quelconque des revendications 9 à 11, comprenant en outre un agent de rhéologie, de préférence un agent épaississant de la phase aqueuse, un agent gélifiant ou un agent suspensif.

13. Composition selon l'une quelconque des revendications 9 à 12 présentant une taille de goutte inférieure ou égale à 30 μm, préférentiellement inférieure ou égale 10 μm.

14. Composition selon l'une quelconque des revendications 9 à 13 présentant une viscosité Brookfield supérieure à 3 000 mPas à 25°C et 20 rpm, de préférence supérieure à 5 000 mPas à 25°C et 20 rpm.

15. Procédé de fabrication d'une composition de type émulsion de Pickering H/E, notamment à usage cosmétique, comprenant les étapes suivantes :

a) la dispersion dans une phase aqueuse d'une composition émulsifiante comprenant au moins une cyclodextrine et un mélange d'au moins un alkyl polyglucoside en C12-C20 et d'au moins un alcool gras linéaire ou ramifié ayant un nombre total d'atome de carbone allant de 8 à 24 , ce dernier étant présent dans un ratio compris entre 0,01 et 1 part, de préférence entre 0,15 et 0,30 part, pour 1 part en poids d'au moins cette cyclodextrine ; ce mélange d'au moins un alkyl polyglucoside en C12-C20 et d'au moins un alcool gras linéaire ou ramifié ayant un nombre total d'atome de carbone allant de 8 à 24 ayant une balance Hydrophile-Lipophile (HLB) supérieure à 8, préférentiellement supérieure ou égale à 9,

b) l'ajout au mélange obtenu à l'étape a) d'une phase grasse, en une quantité comprise entre 10 et 65 % en poids sous une agitation mécanique suffisante pour permettre la dispersion de la phase grasse en fines gouttelettes dans la phase aqueuse et l'obtention d'une émulsion H/E ayant une taille de goutte inférieure ou égale à 30 μm, préférentiellement inférieure ou égale à 10 μm.

## Revendications

1. Composition émulsifiante, notamment à usage cosmétique, apte à permettre l'obtention d'une émulsion de type huile dans eau (H/E), comprenant:

   a. au moins une cyclodextrine,
   b. et au moins un émulsifiant H/E d'origine naturelle, dans laquelle l'émulsifiant H/E est choisi parmi les esters gras de dextrines ou d'amidons hydrolysés, sous forme d'esters octénylsuccinate de sodium, l'émulsifiant H/E présentant une balance Hydrophile-Lipophile (HLB) supérieure à 8, préférentiellement supérieure ou égale à 9.

2. Composition émulsifiante selon la revendication 1, **caractérisée en ce que** l'émulsifiant H/E est présent dans la composition émulsifiante dans un ratio compris entre 0,01 et 1 part, de préférence compris entre 0,05 et 0,5 part, plus préférentiellement compris entre 0,10 et 0,35 part, et mieux encore compris entre 0,15 et 0,30 part, pour 1 part en poids de cyclodextrine.

3. Composition émulsifiante selon la revendication 1 ou 2, dans laquelle ladite au moins une cyclodextrine est choisie parmi les alpha-, beta- et gamma- cyclodextrines, de préférence parmi la beta-cyclodextrine native.

4. Composition émulsifiante selon l'une quelconque des revendications précédentes, dans laquelle la cyclodextrine se présente sous forme d'une poudre cristalline, pseudo-cristalline ou amorphe.

5. Composition émulsifiante selon l'une quelconque des revendications précédentes, dans laquelle l'émulsifiant H/E est choisi également parmi les produits naturellement biodégradables en milieu naturel hydraté avec une balance Hydrophile-Lipophile (HLB) comprise entre 8 et 20, de préférence entre 9 et 16 et mieux encore entre 11 et 14.

6. Composition émulsifiante selon l'une quelconque des revendications 1 à 5 comprenant en outre au moins un polyol ; de préférence choisi parmi le maltitol, le mannitol, le xylitol, l'érythritol, le sorbitol, le glycérol.

7. Composition émulsifiante selon l'une quelconque des revendications 1 à 6 dans laquelle la composition contient, en % en poids :

   a. 40 % à 95 % d'au moins une cyclodextrine,

b. 5 % à 40 % d'au moins un émulsifiant H/E d'origine naturelle, dans laquelle l'émulsifiant H/E est choisi parmi les esters gras de dextrines ou d'amidons hydrolysés, sous forme d'esters octénylsuccinate de sodium, ayant une balance Hydrophile-Lipophile (HLB) supérieure à 8, préférentiellement supérieure ou égale à 9,

c. Et 0% à 40 % d'au moins un polyol.

8. Composition de type émulsion de Pickering H/E, notamment à usage cosmétique, **caractérisée en ce qu'**elle contient pour 1 part en poids d'au moins une cyclodextrine, entre 0,01 et 1 part, de préférence entre 0,15 et 0,30 part d'au moins un émulsifiant H/E d'origine naturelle, dans laquelle l'émulsifiant H/E est choisi parmi les esters gras de dextrines ou d'amidons hydrolysés sous forme d'esters octénylsuccinate de sodium, ayant une balance Hydrophile-Lipophile (HLB) supérieure à 8, préférentiellement supérieure ou égale à 9.

9. Composition selon la revendication 8 contenant une ou plusieurs huiles liquides à température ambiante (25°C), de préférence au moins une huile liquide non volatile.

10. Composition selon la revendication 9, dans laquelle la teneur en huile de l'émulsion H/E finale est comprise entre 10 et 65 % en poids, et de préférence entre 20 et 55 % en poids.

11. Composition selon l'une quelconque des revendications 8 à 10, comprenant en outre un agent de rhéologie, de préférence un agent épaississant de la phase aqueuse, un agent gélifiant ou un agent suspensif.

12. Composition selon l'une quelconque des revendications 8 à 11 présentant une taille de goutte inférieure ou égale à 30 $\mu$m, préférentiellement inférieure ou égale 10 $\mu$m.

13. Procédé de fabrication d'une composition de type émulsion de Pickering H/E, notamment à usage cosmétique, comprenant les étapes suivantes :

a) la dispersion dans une phase aqueuse d'une composition émulsifiante comprenant au moins une cyclodextrine et un mélange d'au moins un émulsifiant H/E d'origine naturelle, dans laquelle l'émulsifiant H/E est choisi parmi les esters gras de dextrines ou d'amidons hydrolysés sous forme d'esters octénylsuccinate de sodium, ce dernier étant présent dans un ratio compris entre 0,01 et 1 part, de préférence entre 0,15 et 0,30 part, pour 1 part en poids d'au moins cette cyclodextrine, et ayant une balance Hydrophile-Lipophile (HLB) supérieure à 8, préférentiellement supérieure ou égale à 9,

b) l'ajout au mélange obtenu à l'étape a) d'une phase grasse, en une quantité comprise entre 10 et 65 % en poids sous une agitation mécanique suffisante pour permettre la dispersion de la phase grasse en fines gouttelettes dans la phase aqueuse et l'obtention d'une émulsion H/E ayant une taille de goutte inférieure ou égale à 30 $\mu$m, préférentiellement inférieure ou égale à 10 $\mu$m.

Figure 1

| Montanov™ 68 (INCI : cetearyl alcohol et cetearyl glucose) | | | | | |
|---|---|---|---|---|---|
| Hydroxyethylcellulose | | | Xanthane | | |
| 5% Béta-cyclodextrines Tournesol | 5% Béta-cyclodextrines Tournesol | 2% Béta-cyclodextrines IPP | 2% Béta-cyclodextrines Tournesol | 2% Béta-cyclodextrines IPP | 5% Béta-cyclodextrines IPP |

Figure 2

Europäisches
Patentamt

European
Patent Office

Office européen
des brevets

**RAPPORT DE RECHERCHE EUROPEENNE**

Numéro de la demande

EP 22 20 9693

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | DATABASE GNPD [Online] MINTEL; 16 janvier 2018 (2018-01-16), anonymous: "Energize Razor", XP093024180, Database accession no. 5381055 * alinéa [ingredients] * ----- | 1-6 | INV. A61K8/06 A61K8/73 A61K8/34 A61K8/37 A61K8/39 A61K8/60 A61Q19/00 |
| X | DATABASE GNPD [Online] MINTEL; 7 mars 2018 (2018-03-07), anonymous: "Brightening Solution Ultra Fine Microfiber Brightening Sheet Mask", XP093024181, Database accession no. 5505805 * alinéa [ingredients] * ----- | 1-6,8-12 | |
| X | DATABASE GNPD [Online] MINTEL; 19 juillet 2013 (2013-07-19), anonymous: "CC Cream SPF 40", XP093024174, Database accession no. 2106614 * alinéa [ingredients] * ----- | 1-6,8-12 | DOMAINES TECHNIQUES RECHERCHES (IPC) A61K A61Q |
| Y | WO 2008/003685 A1 (HENKEL KGAA [DE]; ROTHER MARC PATRICK [DE]; HLOUCHA MATTHIAS [DE]; STR) 10 janvier 2008 (2008-01-10) * revendications 1, 9-10 * * exemples 7-8 * * page 1, ligne 9 – ligne 16 * * page 3, ligne 3 – ligne 17 * * exemple 1 * ----- | 1-13 | |

-/--

Le présent rapport a été établi pour toutes les revendications

| Lieu de la recherche | Date d'achèvement de la recherche | Examinateur |
|---|---|---|
| Munich | 21 février 2023 | Krattinger, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................
& : membre de la même famille, document correspondant

EPO FORM 1503 03.82 (P04C02)

page 1 de 2

RAPPORT DE RECHERCHE EUROPEENNE

Numéro de la demande

EP 22 20 9693

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | MARTO JOANA ET AL: "Pickering emulsions: challenges and opportunities in topical delivery", EXPERT OPINION ON DRUG DELIVERY, INFORMA HEALTHCARE, GB, vol. 13, no. 8, 2 août 2016 (2016-08-02), pages 1093-1107, XP009542220, ISSN: 1742-5247, DOI: 10.1080/17425247.2016.1182489 * tableau 2 * ----- | 1-13 | |
| Y | Marilyn Rayner ET AL: "Quinoa starch granules as stabilizing particles for production of Pickering emulsions", Faraday Discussions, vol. 158, 1 janvier 2012 (2012-01-01), pages 139-155, XP055627992, DOI: 10.1039/c2fd20038d Extrait de l'Internet: URL:https://pubs.rsc.org/en/content/articl epdf/2012/fd/c2fd20038d [extrait le 2019-10-02] * alinéa [01.2] * ----- | 1-13 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 21 février 2023 | Krattinger, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

page 2 de 2

## ANNEXE AU RAPPORT DE RECHERCHE EUROPEENNE
## RELATIF A LA DEMANDE DE BREVET EUROPEEN NO.

EP 22 20 9693

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-02-2023

| Document brevet cité au rapport de recherche | Date de publication | Membre(s) de la famille de brevet(s) | Date de publication |
|---|---|---|---|
| WO 2008003685 A1 | 10-01-2008 | DE 102006031500 A1 | 17-04-2008 |
| | | EP 2091502 A1 | 26-08-2009 |
| | | WO 2008003685 A1 | 10-01-2008 |

EPO FORM P0460

Pour tout renseignement concernant cette annexe : voir Journal Officiel de l'Office européen des brevets, No.12/82

**EP 4 159 187 A1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0685227 A
- FR 2858777
- DE 10239647 A1
- EP 3037083 A1
- FR 2823438 A1
- EP 2091502 B1
- FR 2948285